# EUROPEAN PATENT APPLICATION

(11) **EP 0 943 303 A2**
(43) Date of publication of application: **22.09.1999**
(21) Application number: 99200701.3
(22) Date of filing: 10.03.1999
(51) Int. Cl.: A61F 2/06

(54) **Vascular prothesis and balloon- and catheter assembly for positioning same**

(30) Priority: 16.03.1998 NL 1008599
(71) Applicant: Glastra, Hendrik, 7535 PG Enschede (NL)
(72) Inventor: Glastra, Hendrik, 7535 PG Enschede (NL)
(74) Representative: Timmers, Cornelis Herman Johannes

(57) **Abstract**

1. The invention provides a vascular prothesis (24) for supporting a main bodyvessel (4) at the position of a Y-shaped bifurcation thereof, particularly the bifurcation of the aorta to both the arterie iliaca (6, 8), with a radially expandable mainpart (30) and, extending in Y-shaped fashion therefrom, a first and a second, also radially expandable tubular part (32, 36) connected with the lumen thereof, of which the first tube-part (32) is longer than the second tube-part (36) in such a way that, while bridging an aneurism (14) near the bifurcation of the first branching-off vessel (8) a seal between this tube-part and said vessel can be brought about while the second, short, tube-part cooperates sealingly with a radially expandable stent, extending therein and cooperating sealingly with the second (6) branching-off bodyvessel.

## Description

The invention relates to a vascular prothesis for the local support of a main bodyvessel and of a first and a second, vessel, Y-shaped branching therefrom, said prothesis comprising a radially expandable mainpart with, Y-shaped extending therefrom, a first and a second, radially expandable tube-part being in connection with the lumen thereof, said first tube-part being longer than the second part to sealingly bridge an aneurism of the first vessel.

A prothesis of this kind is known from WO 95/13033. This known prothesis is intended to be used when the patient has an aneurism of the main bodyvessel only and possibly also of only one of the vessels branching therefrom; the short tube-part is intended only to facilitate the positioning of the prothesis.

A vascular prothesis, for supporting an aorta-aneurism at the position of the bifurcating of the left- and right respectively arterie iliaca, is known in itself from EP-A-0 686 379. In this known prothesis from the central, radially expandable mainpart extend two short, also radially expandable tube-parts in each of which a radially expandable stent must be placed which, after its radial expansion, must come to lie sealingly against the supporting tube-part and also against the innerwall of the relative arterie iliaca.

The positioning of such a prothesis implies that first by the central mainpart must be positioned at he correct position and then must be expanded radially (which can be done by introducing the mainpart in compressed state and letting it thereafter expand radially by its own elasticity or by using an expandable balloon) whereafter, via de first and the second arterie iliaca the stent, which is to be connected thereto, must put into place by means of a catheter. Not only is this treatment time-consuming and thus a burden for the patient, but there is also the risk of leaks at the respective connecting points between the tube-shaped stents and the short tube-stumps which receive them of the central mainpart.

WO 96/29955 describes a radially expandable vascular prothesis with a first, elongate part, extending from the aorta past the bifurcation into the first branching-off vessel and with an opening in its sidewall, which must sealingly accept the end of a second elongate part extending into the second branching-off vessel. The prothesis parts are of a special nature with a number of a versions along their length, and a correct seal between the second elongate part and the boundery of the opening is difficult, if not impossible, to achieve.

The invention aims to eliminate the abovementioned drawbacks of the prior art and thereto proposes that the first tube-part is longer than the second tube-part, in such a way that, while bridging an aneurism, if any, of the first branching-off vessel near to the bifurcation, a seal can be brought about between this tube-part and the branching-off bodyvessel, while the second, short, tube-part cooperates sealingly with a radially expandable stent, which extends therein and cooperates sealingly with the second branching-off bodyvessel.

For the positioning of this prothesis the central mainpart is firstly displaced so far into the bodyvessel that the second, short, tube-part is brought past the bifurcation whereafter, by a backward movement of the central mainpart, the second tube-part is brought into the second bodyvessel so that, after radially expanding of the mainpart and both the tube-parts extending therefrom, the first bodyvessel already contains a sufficiently long reinforcing stent part. It is then only necessary to put into place, via the second bodyvessel, the second stent to complete the prothesis.

Preferably the positioning of the second stent will be done by using a catheter-balloon assembly such as described in EP-0 779 062 by means of which an excellent sealing connection of the second stent to the short tubular part is obtained.

Further preferred embodiments are described in the claims 2-5.

The claimed exclusive rights include a catheter assembly to be used in combination with the abovementioned prothesis assembly, such as described in claim 6.

The invention will be elucidated on the hand of the drawing. Therein shows:
fig. 1 a cross section through the lower part of an aorta with, extending therefrom, the left and right arterie iliaca;
fig. 2 a longitudinal cross section through the vascular prothesis as proposed by the invention;
figs. 3-7 cross sections which elucidate the positioning of the stent according to the invention.

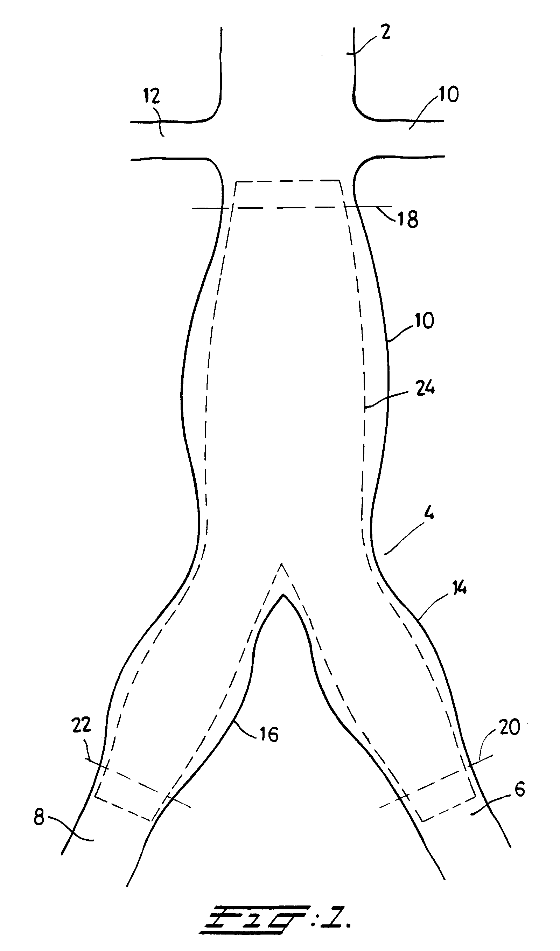

Fig. 1 shows the lower part of an aorta 2 in which there is, a short distance before the bifurcation 4 from which extend the righthand arterie iliaca 6 and the lefthand arterie iliaca 8 respectively, an aneurism (widening of the vesselwall) 10. As to the bloodvessels leading to the kidneys: the righthand arterie renata is denoted with 10; the lefthand with 12.

The righthand and lefthand arterie iliaca respectively, also have an aneurism indicated with 14 and 16 respectively. Practice has shown that an aorta aneurism is nearly always accompanied with an aneurism in the upper part of the righthand and lefthand respectively arterie iliaca.

The prothesis which is to be introduced must thus bridge the aneurism 10 as well as the aneurism 14 and 16, and this in such a way that at the upper end of the prothesis a sealing connection is obtained directly below the both arterie renata 10 and 12 respectively, thus the place which is indicated in fig. 1 with the dashline 18. The sealing connection in both the lower bloodvessels, 6 and 8, must be present past the both aneurisms 14 and 16 thus on the positions which in fig. 1 denoted with the dashlines 20 and 22 respectively. Such a prothesis must thus, eventually have the shape which is in the figure denoted with the dashlines and indicated with reference number 24; it is literally of vital importance that there is a complete sealing between the prothesis on the one hand and the respective bodyvessels, thus at the positions 18, 20 and 22; when there is at any place a leak the volume between the prothesis and the vesselwall is subjected to the bloodpressure and thus in the long run the treatment will not be effective. This implies that preferably also within the prothesis there must be the smallest possible number of interconnections between the parts of the prothesis.

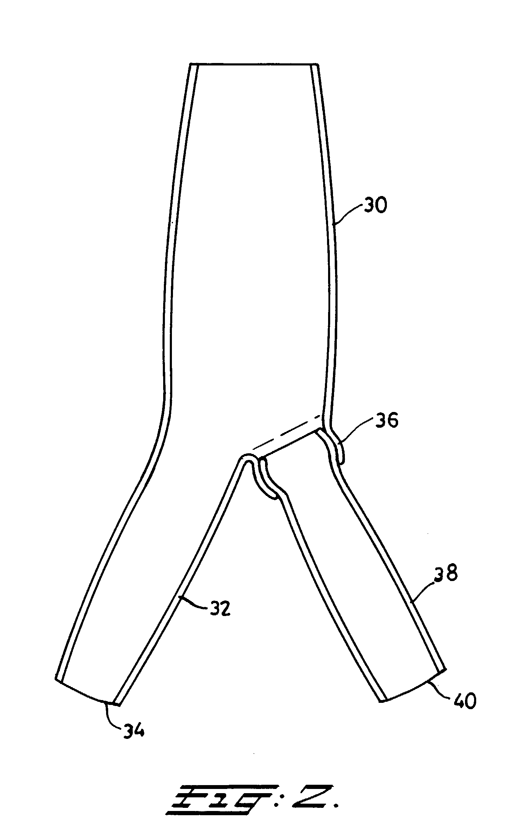

The prothesis proposed according to the invention is shown in crosssection in fig. 2. This prothesis has a first, essentially tubular mainpart 30, preferably made from radially expandable and curing material, with, in one piece therewith, an elongate tubular part or "tube" 32. This "tube" has such a length that when the prothesis is put into place - see fig. 1 - the end 34 thereof reaches past the stenosis 16, thus at the position 22 where the sealing connection with the arterie iliaca 8 must be obtained. Furthermore from this mainpart 30 extends a second tube 36 but this is only short; its purpose will be described later on. This tube 36 cooperates with a tubular stent 38 which is, as well as the other parts of the prothesis, made from radially expandable and preferably curable material and this tubular stent 38 has such a length that when the prothesis is put into place the end 40 thereof reaches past the aneurism 14, thus lies at the position 20 where the sealing against the vessel 6 must take place.

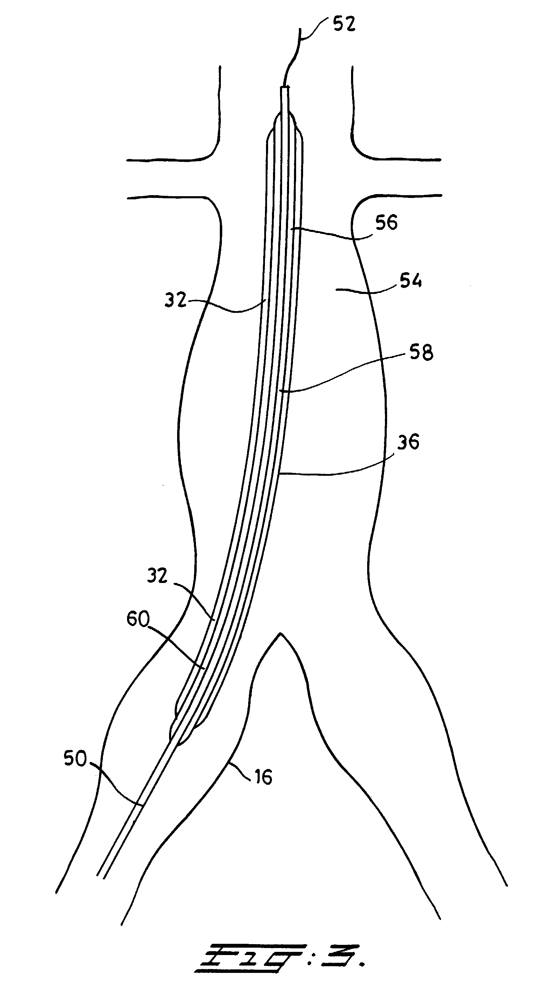

The purpose of the configuration proposed by the invention and the way in which it is introduced will be elucidated on the hand of the figs. 3-7.

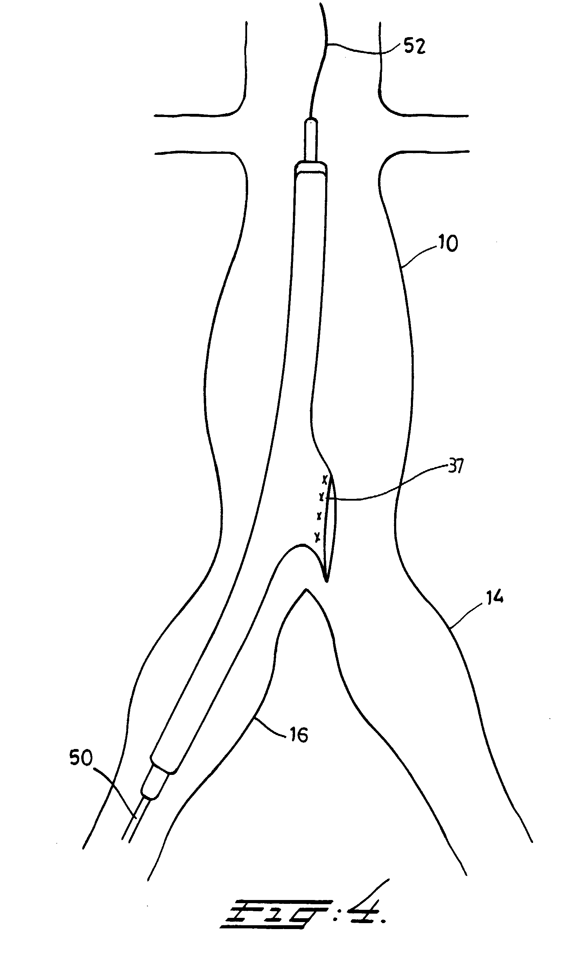

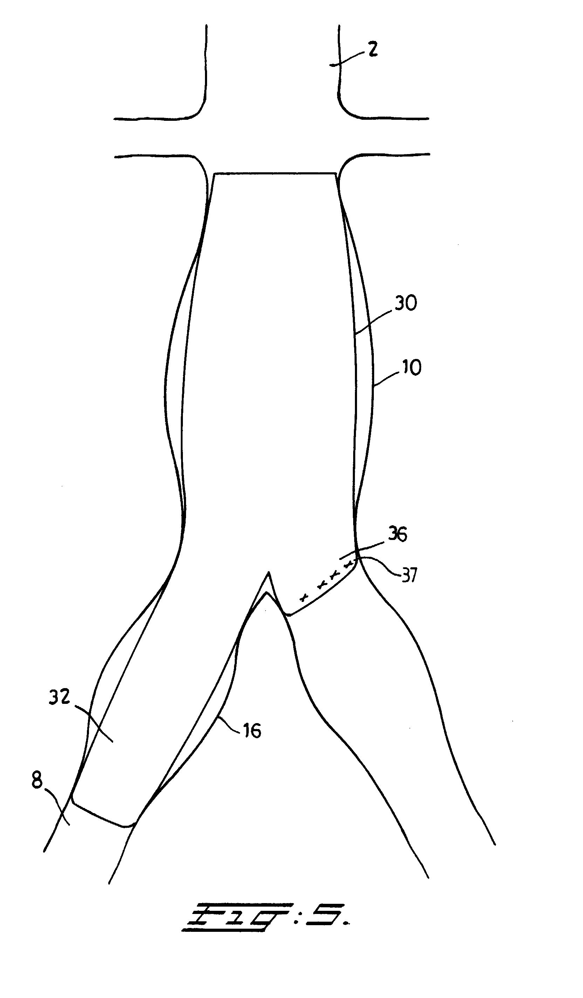

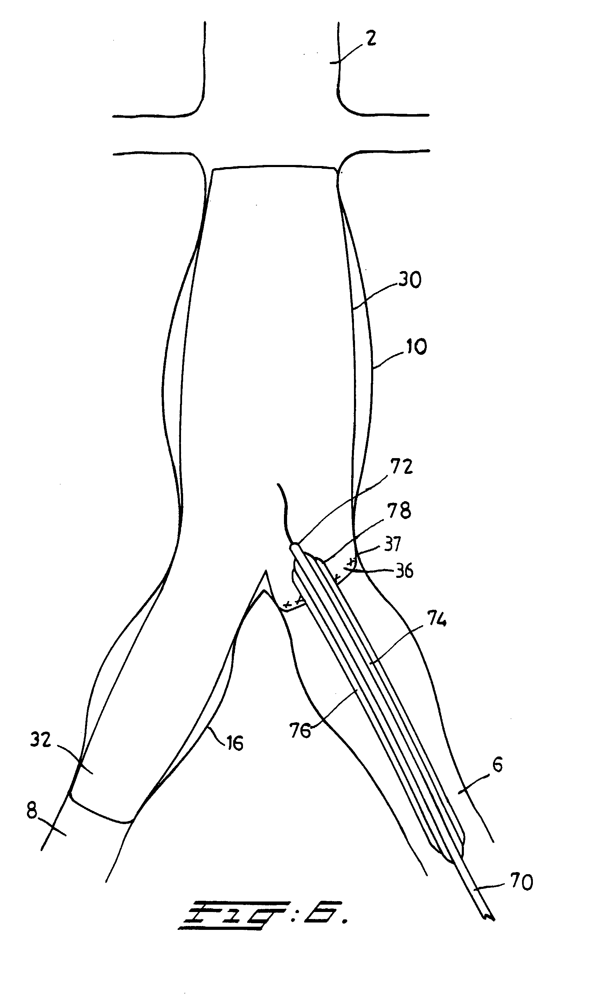

Firstly via the lefthand arterie iliaca 16 an assembly is introduced which comprises a catheter 50 with guidewire 52 and carrying at the distal end 54 thereof an expandable balloon 65, the lumen of which may be connected to some of a short auxiliary balloon (not shown). Around this balloon there is a radially expandable prothesis from curable material which has in expanded state the configuration according to fig. 2, thus with the mainpart 30, the long "tube" 32 and the short "tube" 36. This not yet cured and thus flexible assembly, is brought in the position according to fig. 3 and thereafter shifted back in such a way that the situation according to fig. 4 is obtained. When the tubular balloon 65 is expanded in the usual way by supplying a suitable medium under pressure thereto the situation according to fig. 5 is obtained: the balloon has the mainpart 30 of the prothesis in the desired way pressing against the innerwall of the aorta 2 and the long "tube" 22 lies against the innerwall of the arterie iliaca 8, bridging the aneurism 10 and the aneurism 16. Preferably one will use a prothesis which is made from a material which cures under the influence of radiation and of which the curing is, as common and known from the prior art, initiated by a short illumination of this material, so that after some time and after the removal of the catheter and the balloon the structure according to fig. 5 is obtained. The short "tube" 30 will receive only little radiation or none at all and thus remains flexible; furthermore the bloodstream will keep the short "tube" open. It is possible to choose the material of the stent in this part in such a way that the curing process thereof occurs initially not so fast as in the material of the remaining part of the prothesis. In this way the structure can be completed - see fig. 6 - by introducing via the righthand arterie iliaca 6 the catheter 70 with guidewire 72 carrying at its distal end the expandable balloon 74 with around it the - also known - radially expandable and curable stent 76.

To facilitate the correct positioning of the stent 76 in the short "tube" 36 both the stent 76 and the tube 36 carry on their circumference a ring of x-ray opaque markers 37 and 77 respectively.

By expanding the balloon by supplying thereto fluidum under pressure the stent 76 is expanded radially; this stent, too, is made from a material of which the curing is initiated by a short radiation, such as known in itself. The upper end 78 of this stent 76, which lies near the distal end of the catheter 70 will lie sealingly against the innerwall of the tube 36 for which, as shown, one will preferably use the configuration as described in EP-0 779 062.

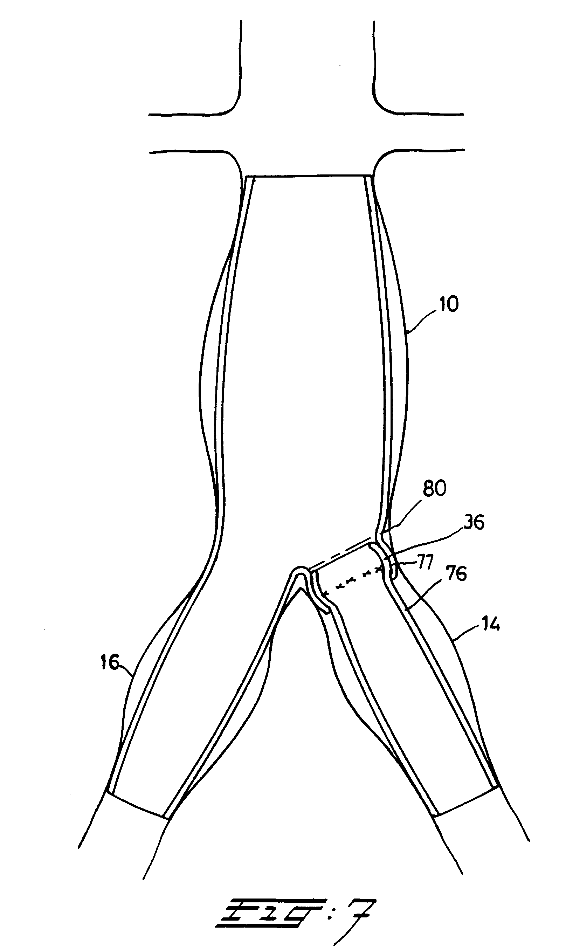

After the withdrawal of the catheter 70 the situation of fig. 7 is obtained. All three aneurisms 10, 14, 16 are bridged and the only position in the complete prothesis in which parts must lie sealingly against each other is at the level 80, thus where the tubular stent 76 closely connects to the short "tube" 36.

## Claims

1. Vascular prothesis (24) for the local support of a main bodyvessel (4) and of a first and a second, vessel, Y-shaped (8, 61) branching therefrom, comprising a radially expandable mainpart (30) with, Y-shaped extending therefrom, a first (32) and a second (36), radially expandable tube-part being in connection with the lumen thereof, said first tube-part (32) being longer than the second tube-part (36), to sealingly bridge an aneurism of the first vessel, characterized in that the second, short, tube-part (36) cooperates sealingly with a radially expandable stent (38), extending therein, to bridge an aneurism of the second vessel and cooperating sealingly with the second branching-off bodyvessel (6).

2. Prothesis according to claim 1 characterized in that the component parts thereof comprise a curing plastics of which the curing process can be initiated by the irradiation thereof with radiation with a short wavelength.

3. Prothesis according tot claim 2 characterized in that the end of the stent (38) to be placed into the second branching-off bodyvessel (8) and which extents in to the second tube-part is trumpet-shaped and lies against a corresponding enlargement (36) of the second tube-part.

4. Prothesis according to claims 2-3 characterized in that the second tube-part consist of a material of which the curing takes place at a slower rate than the curing of the material of the remaining part of the prothesis.

5. Prothesis according to claims 3-4, characterized in that the short tube-part carries around the circumferences thereof X-ray opaque markers (37) while the end of the stent to be inserted therein carries corresponding X-ray opaque markers (77).

6. Catheter-balloon assembly (50, 52) to be used for positioning a prothesis according to claims 1-5, comprising a main catheterpart with at the distal end (54) thereof an inflatable main balloon (65), and extending therefrom a first auxiliary balloon for supporting the longer tube-part and, branching-off from said distal end a second auxiliary balloon for supporting the second, short, tube-part.
